# EUROPEAN PATENT SPECIFICATION

(11) **EP 4 450 001 B1**
(45) Date of publication and mention of the grant of the patent: **30.09.2026**
(21) Application number: 24162859.3
(22) Date of filing: 12.03.2024
(51) Int. Cl.: A61B 17/068

(54) **FEEDER FOR IMPLEMENTATION OF COMPRESSION CLAMP FOR ORTHOPAEDIC ANASTOMOSES AND THE KIT CONTAINING IT**
ZUFÜHRVORRICHTUNG ZUR DURCHFÜHRUNG EINER KOMPRESSIONSKLEMME FÜR ORTHOPÄDISCHE ANASTOMOSEN UND KIT DAMIT
DISPOSITIF D'ALIMENTATION POUR LA MISE EN OEUVRE D'UNE PINCE DE COMPRESSION POUR ANASTOMOSES ORTHOPÉDIQUES ET KIT LE CONTENANT

(30) Priority: 30.03.2023 PL 44424423
(43) Date of publication of application: 23.10.2024
(73) Proprietor: Pomorski Uniwersytet Medyczny w Szczecinie, 70-204 Szczecin (PL)
(72) Inventor: Kolodziej, Lukasz, 71 497 Szczecin (PL)
(74) Representative: Kondrat, Mariusz

(56) References cited:
- KR-A- 20220 030 705
- US-A1- 2008 140 091
- US-A1- 2008 269 727
- US-A1- 2022 361 877
- US-B1- 9 168 050
- US-B2- 10 368 897

## Description

The object of the invention is a feeder for implementation of compression clamps for orthopaedic anastomoses and a kit containing it.

Surgical clamps are widely used in medical applications. From document US6638297 B1, a medical clamp feeder comprising a handle part, rotating elements, a shank part, an anvil part and a cartridge assembly is known. A knife (not shown) is slidable within the cartridge assembly to cut tissue. The handle section has a first trigger or closing trigger (also known as the locking trigger) and a second release trigger. The closing trigger causes the anvil part to approach the cartridge assembly. The release trigger causes the clamps to slide out of the cartridge and form on the anvil. The trigger also causes the blade to move through the cartridge assembly to cut through the tissue. Further, the document under discussion discloses a surgical abutment, in the form of a clamp having a crown having a first and a second end and a longitudinal axis extending therebetween. The clamp also includes first and second legs, which are provided with first ends attached to the first and second ends of the crown. Said legs also have second ends with preferably pointed ends, said second ends extending from the crown in a direction generally perpendicular to the longitudinal axis.

Commercially available medical compression clamps require Nitinol - a shape memory material containing at least 20 % nickel additive - i.e. an allergenic material, in order to function effectively. **In** addition, a nitinol clamp, which is initially mechanically opened, constantly strives to return to its original, tapered shape after implantation. This results in a strong pressure of the clamp arms on the bone tissue and potentially "bone receding and atrophying" under the strong pressure, and therefore a loss of the original correction (proper alignment of the fragments) and a potential lack of fusion of the fused bones.

American patent application US2022361877A1 relates to a staple instrument for inserting and removing staples from bone that is equipped with a rotational drive shaft providing finite adjustments to open and close jaws used to hold and adjust the legs of a staple. When turning the handle clockwise, the drive shaft drives the nut away from the handle, which causes the linkage mechanism to spread and expand the staple holder. The shaft is segmented and equipped with a threading and a nut. The movable jaws are segmented and terminated with staple hooks that remain generally parallel with the bridge of the staple so that the instrument can insert the staple to a maximum depth into the bone.

American patent application US2008140091 A1 relates to a minimally invasive device for suture repair of soft tissue comprising an elongated housing having a proximal end and a distal end. Said distal end comprises a suturing assembly comprising an upper jaw, a lower jaw and a flexible needle adapted to carry a suture. Wherein the upper and lower jaws are pivotally mounted with respect to each other and with respect to the housing and the needle being movable between a first position and a second position and wherein the needle is substantially housed within and extendable from and retractable within one of the jaws and the housing and extends to the proximal end of the housing through the housing. Proximal end of housing comprises a handle, a needle actuator and a jaws actuator, the needle actuator connected to the needle for extension and retraction of the needle from the needle-containing jaw, and the jaws actuator linked to the jaws within the housing for closing and opening of the jaws with respect to each other and for grasping and releasing the tissue to be sutured.

American patent US9168050 B1 relates to a medical instrument tool or end effector constructed of a fixed jaw having a distal grasping end and a proximal body end; a movable jaw having a distal grasping end and a proximal body end; a slide member controlled from a tool actuator, adapted for linear translation and supported between the jaws; at least one link that inter-couples between the slide member and the movable jaw; and a pivot member mounted at the fixed jaw and for engagement with a slot in the body end of the movable jaw. In the disclosed apparatus, the upper jaw is enclosed in the channel of the main body of lower jaw, wherein at least two links are required to attach the upper jaw to lower jaw. By pushing the handle further into the cannula or port of entry the surgeon can then place the open jaws in position to close about the target object. The surgeon can then use the jaw clamping means to close the jaws on the target object by squeezing the jaw actuation lever. At least upper jaw is equipped with multiple serrations for enhanced the gripping of tissue. In favourable embodiment both jaws have opposing serrations.

The purpose of the invention is to provide a feeder and a kit for fixing compression clamps for orthopaedic anastomoses, ensuring that the clamp is fixed in a way that does not cause uncontrolled compression of the edges of the anastomosed bones, which may consequently lead to damage to the bones.

The essence of the invention is a feeder for implementation of compression clamps for orthopaedic anastomoses according to claim 1.

Preferably, the hole spacing of the sight block is wider than the arm spacing of the compression clamp by 0.5-0.7 mm.

Preferably, the closing lever is fitted with a bent end.

Preferably, the deflection of the bent end is 20° with respect to the longitudinal axis of the feeder.

Preferably, the handle is equipped with an anti-rotational flattening.

Preferably, the handle is finished with a plate.

Another subject of the invention is a compression clamp kit for orthopaedic anastomoses according to claim 7.

Preferably, the compression clamp is composed of a low-profile base and vertical arms.

Preferably, vertical arms have pointed ends.

The invention provides the following benefits:
- The insertion of the clamp arms, which are narrower in relation to the holes drilled by the dedicated sight, results in immediate mechanical compression of the bony fragments;
- The feeder and kit according to the invention ensures a better fit of these clamped bone fragments without uncontrolled 'giving way and bone atrophy' under excessive pressure, ensuring proper alignment of the fragments and proper fusion of the fused bones;
- Attaching the sight to the feeder at an angle (preferably 30°) provides a better view of the operating field during bone drilling than a vertical attachment;
- The feeder is ergonomic and comfortable to use.

The invention is illustrated in an example of execution and in the drawing in which fig. 1 shows: the feeder according to the invention in top view; fig. 2 shows a side view of the feeder according to the invention in a configuration with the closing lever closed; fig. 3 shows a side view of a feeder according to the invention in a configuration with the closing lever raised, where detail A shows close-up of the jaws of the distal part of the feeder with particular reference to the location and shape of the groove; fig. 4 shows a side view of a feeder according to the invention in a locked configuration with a closed closing lever with an attached sight; fig. 5 shows a side view of a feeder according to the invention in a locked configuration with a closed closing lever with the clamp attached; fig. 6 shows a front view of the feeder sight according to the invention (A) and in side view (B); while fig. 7 shows a clamp included in the kit according to the invention; fig. 8 shows a clamp from the kit according to the invention during insertion into holes drilled in the bones using a dedicated feeder sight according to the invention and are spaced 0.5 mm wider than the width of the arms of the clamp; fig. 9 shows a clamp from the kit according to the invention mounted in the target position.

### Example 1.

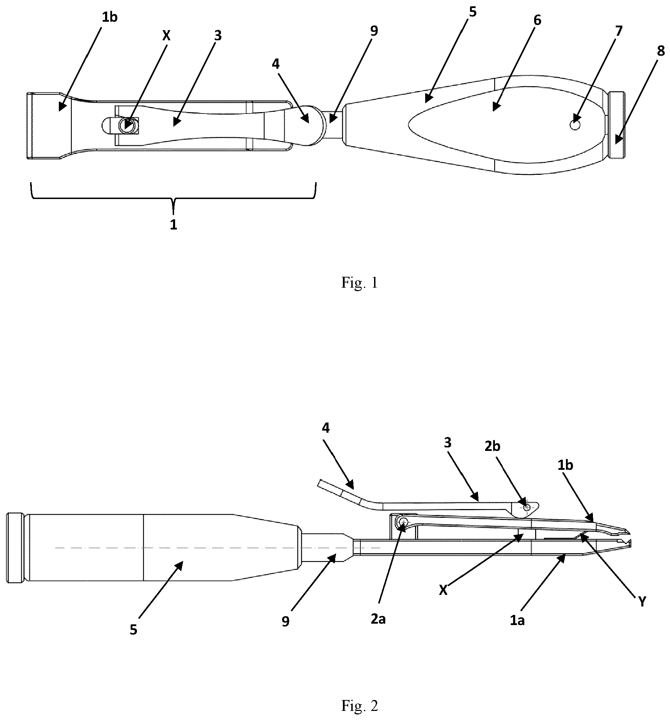

A feeder for implementation of compression clamps for orthopaedic anastomoses according to the invention is shown in figs. 1-4, wherein **1** the distal part; **1a** is the lower jaw; **1b** is the upper jaw; **2a** is the first fixing pin; **2b** is the second fixing pin; **3** is the closing lever; **4** is the bent end; **5** is the handle; **6** is the flattening of the handle; **7** is the double-sided pin; **8** is the plate; **9** is the shaft; **10** is the groove; **11** is the sight; **12** is the compression clamp; **X** is the cylinder; **Y** is the spring plate.

As indicated in fig. 1 the feeder according to the invention consists of a distal part **1,** a shaft part **9** and a handle **5.** The attachment of the handle **5** to the shaft **9** has been secured by a double-sided connecting pin **7,** which is a bonding element. In this example implementation, the double-sided connecting pin **7** is made of stainless steel, while the handle **5** is made of medical grade resin Radel^{™} R-5500.

The handle **5** of the feeder according to the invention, used to hold the feeder in the hand of the user, is provided with an anti-rotational flattening of the handle **6,** which provides ergonomics and comfort in use. Furthermore, in this example of implementation, the handle **5** is provided with a plate **8,** which forms the end of the handle **5.** The plate **8** serves to be struck with a hammer when the compression clamp **12** is fixed in the target position and prevents damage to the handle **5.** In this example of implementation, the plate **8** is made of stainless steel.

As indicated in figs. 1-2, the distal part 1 of the feeder according to the invention comprises an upper jaw **1b** movably connected by a first fixing pin **2a** to a stationary lower jaw **1a** and by a second fixing pin **2b** to a closing lever **3.** In this example of execution, the lower jaw **1a** and the upper jaw **1b** are made of stainless steel, the first fixing pin **2a** and the second fixing pin **2b** are made of stainless steel, while the closing lever **3** is made of titanium. As indicated in figs. 1-2, permanently connected to the lower jaw **1a** is a cylinder **X** made of stainless steel, which provides a guide (up and down) for the upper jaw **1b.** At the top of the cylinder **X** is a mounting for the closing lever **3** by means of a second fixing pin **2b.** In addition, there is a stainless steel spring plate **Y** between the lower jaw **1a** and the upper jaw **1b,** which ensures that the arms open again, i.e. the upper jaw **1b** is lifted when the closing lever **3** is released.

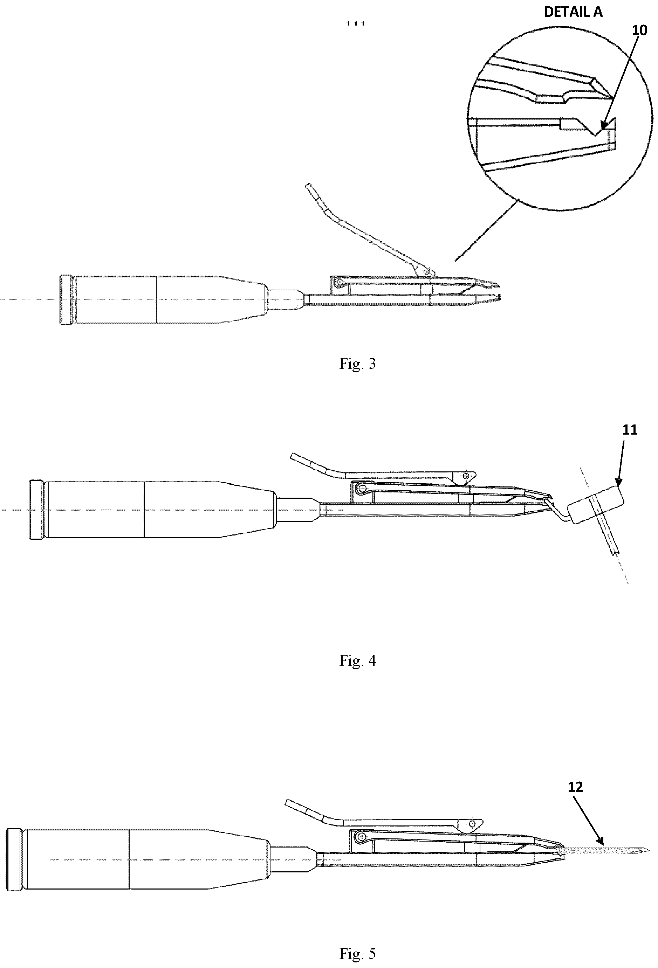

Whereby, the closing lever **3** in the raised position (fig. 3) releases the grip of the lower jaw **1a** and the upper jaw **1b,** while in the lowered position (as in fig. 2 and figs. 4-5) it presses the jaws **1a** and **1b** of the feeder. In this example of execution, the closing lever is provided with a bent end **4** (fig. 1-2), which facilitates its lifting during use of the feeder according to the invention. In this example of execution, the deflection of the bent end **4** is 20° with respect to the longitudinal axis of the feeder indicated by the dotted line in fig. 2.

As indicated in fig. 3, the shaft **9** is connected to a stationary lower jaw **1a**. Whereby, the lower jaw **1a** is provided with a substantially triangular groove **10,** which is shown in detail in fig. 3. The aforementioned groove **10** is designed for the detachable attachment of either the holder **D** of the sight **11** (as shown in fig. 4) or the compression clamp **12** (as shown in fig. 5).

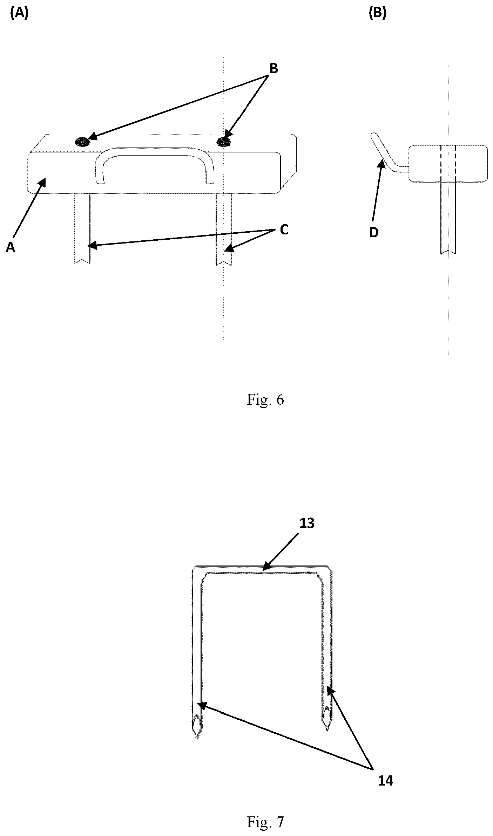

Whereby, the sight is shown in detail in fig. 6, wherein **A** denotes the block of the sight **11; B** denotes holes; C denotes drill guides; **D** denotes holder. As indicated in fig. 6, the sight **11** comprises a sight block A, which is its essentially rectangular body, which for example can be made of stainless steel.

On the upper surface of the block **A** of the sight **11** are located two holes **B.** The holes **B** are through-holes in which drill guides **C** made of stainless steel, for example, are located.

On the other hand, along the long side of the sight, a holder **D** is designed for fixing the sight **11** in the groove **10.** Whereby, as indicated in fig. 4 and fig. 6, the holder **D** enables the sight **11** to be positioned in the groove **10** at an angle of 30°, which provides a better view of the operating field during the drilling of the bone than a vertical fixation. With that said, the spacing of the holes **B** in the block **A** of the sight **11** is designed for a specific size of the compression clamp **12.** The spacing of the holes **B** is wider than the shoulder spacing of the compression clamp **12** for orthopaedic anastomoses, which is to be fixed in the groove **10** of the tray after the sight **11** has been removed. In this manufacturing example, the spacing of the holes **B** is 0.5 mm wider than the spacing of the compression clamp **12.**

### Example 2.

The feeder as in example 1, except that spacing of the holes **B** is 0.7 mm wider than the spacing of the compression clamp **12.**

### Example 3.

A kit for fixing compression clamps for orthopaedic anastomoses according to the invention is shown in fig. 5. In this manufacturing example, the set comprises a feeder according to example 1 and a compression clamp **12,** which is detachably fixed in a groove **10** interchangeably with a sight **11.** As indicated in fig. 7, the substantially C-shaped compression clamp **12** is composed of a low-profile base **13** and vertical arms **14** which have pointed ends. Whereby, in this example implementation, the spacing of the vertical arms **14** of the compression clamp **12** is narrower by 0.5 mm than the spacing of the holes **B** on the block **A** of the sight **11.**

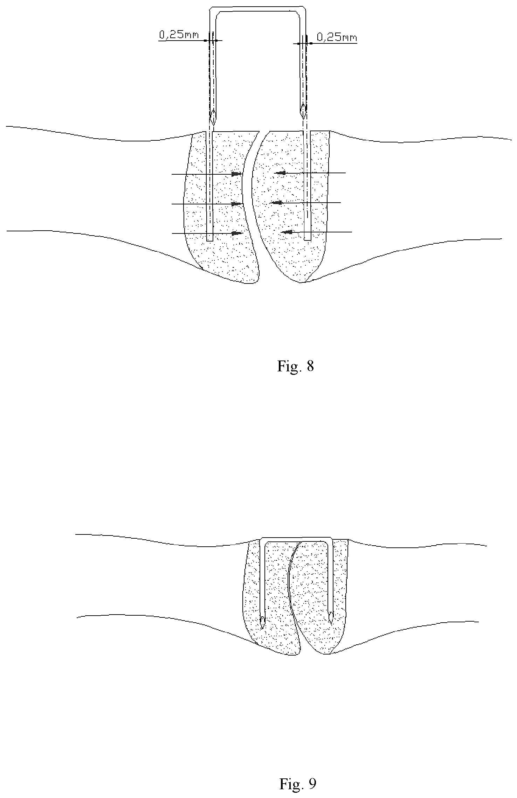

However, during implantation, the bone fragments, as in any other typical joint fusion/arthrodesis technique, should be pre-compressed and temporarily stabilised using manual compression (manually by an assistant) or surgical instruments. In contrast, the actual method of fixing a compression clamp into the patient's bone involves the following steps:
a) location of the sight **11** in the groove **10** of the feeder according to the invention;
b) closing of closing lever **3,** which clamps the jaws **1a** and **1b** of the feeder (fig. 4);
c) symmetrical positioning of the sighting arms **11** fixed in the jaws **1a** and **1b** feeder according to the invention on both sides of the fused bone fragments;
d) inserting the drill into the first hole **B** of the sight **11** and drilling a channel through the entire width of the bone;
e) drilling of the bone canal through the second hole **B** of the sight **11;**
f) after completion of drilling, the sight **11** is disconnected from the feeder according to the invention
   by lifting the closing lever **3;**
g) placing the compression clamp **12** o in the groove **10** and closing the closing lever **3** (as shown in fig. 5);
h) insertion of a compression clamp with a spacing 0.5 mm narrower than the width of the created channels (as schematically illustrated in fig. 8) into the resulting arm holes;
i) the flattened shoulder of the clamp against the surface of the fused bone fragments for with a mallet or hammer.

The insertion of the clamp arms, which are narrower in relation to the holes drilled by the dedicated sight, results in immediate mechanical compression of the bone fragments as shown in fig. 9.

### Example 4.

The kit as in example 3, except that the spacing of the vertical arms **14** of the compression clamp **12** is 0.7 mm narrower than the spacing of hole **B** on block **A** of sight **11.**

## Claims

1. A feeder for the implementation of compression clamps for orthopaedic anastomoses comprising a distal part (1), a shaft part (9) and a handle (5), wherein the distal part (1) consists of a pair of jaws (1a, 1b) and a closing lever (3); wherein an upper jaw (1a) is movably connected by a first fixing pin (2a) to a fixed lower jaw (1b) and the closing lever (3) is movably connected by a second fixing pin (2b) to the lower jaw (1b) through a cylinder (X), that is permanently connected to the lower jaw (1a) and provides an up and down movement guide for the upper jaw (1b); a mounting for the closing lever (3) by means of the second fixing pin (2b) is located at the top of the cylinder (X); a jaws-release mechanism in the form of a spring plate (Y) is located between the lower jaw (1a) and the upper jaw (1b); the lower jaw (1a) is provided with a single groove (10) for detachably fixing a holder (D) of a sight (11), a block (A) of which is provided with holes (B) in which guides (C) are placed; wherein the spacing of the holes (B) of the block (A) of the sight (11) being wider than the arms spacing of a compression clamp (12).

2. The feeder according to claim 1, **characterised in that** the hole spacing (B) of the block (A) of the sight (11) is 0.5-0.7 mm wider than a shoulder width of the compression clamp (12).

3. The feeder according to claims 1 or 2, **characterised in that** the closing lever (3) is provided with a bent end (4) opposite to the second fixing pin (2b).

4. The feeder according to claim 3, **characterised in that the** deflection of the bent end (4) is 20° with respect to the longitudinal axis of the feeder when the closing lever (3) is in a closed position.

5. The feeder according to any of the preceding claims 1 to 4, **characterised in that** a handle (5) is provided with a flat portion (6).

6. The feeder according to any of the preceding claims 1 to 5, **characterised in that** the handle (5) is terminated by a plate (8).

7. A compression clamp kit for orthopaedic anastomoses comprising a feeder for implementation of compression clamps according to any of the preceding claims 1 to 6, and a compression clamp (12) that is detachably fixed in the groove (10) interchangeably with the sight (11).

8. The kit according to claim 7, **characterised in that** the compression clamp (12) is composed of a low-profile base (13) and vertical arms (14).

9. The kit according to claim 8, **characterised in that** the vertical arms (14) have pointed ends.

## Patentansprüche

1. Eine Zuführvorrichtung für die Implementierung von Kompressionsklammern für orthopädische Anastomosen, umfassend einen distalen Teil (1), einen Schaftteil (9) und einen Griff (5), wobei der distale Teil (1) aus einem Paar von Backen (1a, 1b) und einem Schließhebel (3) besteht; wobei eine obere Backe (1a) durch einen ersten Befestigungsstift (2a) mit einer festen unteren Backe (1b) beweglich verbunden ist und der Schließhebel (3) durch einen zweiten Befestigungsstift (2b) mit der unteren Backe (1b) durch einen Zylinder (X) beweglich verbunden ist, der dauerhaft mit der unteren Backe (1a) verbunden ist und eine Auf- und Abwärtsbewegungsführung für die obere Backe (1b) bereitstellt; wobei sich eine Halterung für den Schließhebel (3) mittels des zweiten Befestigungsstifts (2b) an der Oberseite des Zylinders (X) befindet; wobei sich ein Backenlösemechanismus in Form einer Federplatte (Y) zwischen der unteren Backe (1a) und der oberen Backe (1b) befindet; wobei die untere Backe (1a) mit einer einzelnen Nut (10) zur lösbaren Befestigung eines Halters (D) einer Zielvorrichtung (11) versehen ist, deren Block (A) mit Löchern (B) versehen ist, in denen Führungen (C) angeordnet sind; wobei der Abstand der Löcher (B) des Blocks (A) der Zielvorrichtung (11) größer als der Armabstand einer Kompressionsklammer (12) ist.

2. Die Zuführvorrichtung nach Anspruch 1, **dadurch gekennzeichnet, dass** der Lochabstand (B) des Blocks (A) der Zielvorrichtung (11) um 0,5-0,7 mm größer als eine Schulterbreite der Kompressionsklammer (12) ist.

3. Die Zuführvorrichtung nach Anspruch 1 oder 2, **dadurch gekennzeichnet, dass** der Schließhebel (3) gegenüber dem zweiten Befestigungsstift (2b) mit einem gebogenen Ende (4) versehen ist.

4. Die Zuführvorrichtung nach Anspruch 3, **dadurch gekennzeichnet, dass** die Auslenkung des gebogenen Endes (4) in Bezug auf die Längsachse der Zuführvorrichtung 20° beträgt, wenn sich der Schließhebel (3) in einer geschlossenen Position befindet.

5. Die Zuführvorrichtung nach einem der vorhergehenden Ansprüche 1 bis 4, **dadurch gekennzeichnet, dass** der Griff (5) mit einem flachen Abschnitt (6) versehen ist.

6. Die Zuführvorrichtung nach einem der vorhergehenden Ansprüche 1 bis 5, **dadurch gekennzeichnet, dass** der Griff (5) in einer Platte (8) endet.

7. Ein Kompressionsklammer-Set für orthopädische Anastomosen, umfassend eine Zuführvorrichtung für die Implementierung von Kompressionsklammern nach einem der vorhergehenden Ansprüche 1 bis 6 und eine Kompressionsklammer (12), die im Austausch mit der Zielvorrichtung (11) lösbar in der Nut (10) befestigt ist.

8. Das Set nach Anspruch 7, **dadurch gekennzeichnet, dass** die Kompressionsklammer (12) aus einer flachen Basis (13) und vertikalen Armen (14) besteht.

9. Das Set nach Anspruch 8, **dadurch gekennzeichnet, dass** die vertikalen Arme (14) spitze Enden aufweisen.

## Revendications

1. Un applicateur pour la mise en place de pinces de compression pour anastomoses orthopédiques, comprenant une partie distale (1), une partie de tige (9) et une poignée (5), dans lequel la partie distale (1) consiste en une paire de mâchoires (1a, 1b) et un levier de fermeture (3) ; dans lequel une mâchoire supérieure (1a) est reliée de manière mobile par une première broche de fixation (2a) à une mâchoire inférieure fixe (1b) et le levier de fermeture (3) est relié de manière mobile par une seconde broche de fixation (2b) à la mâchoire inférieure (1b) par l'intermédiaire d'un cylindre (X), qui est relié de manière permanente à la mâchoire inférieure (1a) et fournit un guide de mouvement de haut en bas pour la mâchoire supérieure (1b) ; un montage pour le levier de fermeture (3) au moyen de la seconde broche de fixation (2b) est situé au sommet du cylindre (X) ; un mécanisme de libération des mâchoires sous la forme d'une plaque à ressort (Y) est situé entre la mâchoire inférieure (1a) et la mâchoire supérieure (1b) ; la mâchoire inférieure (1a) est pourvue d'une seule rainure (10) pour fixer de manière amovible un support (D) d'un viseur (11), dont un bloc (A) est pourvu de trous (B) dans lesquels des guides (C) sont placés ; dans lequel l'espacement des trous (B) du bloc (A) du viseur (11) est plus grand que l'espacement des bras d'une pince de compression (12).

2. L'applicateur selon la revendication 1, **caractérisé en ce que** l'espacement des trous (B) du bloc (A) du viseur (11) est supérieur de 0,5 à 0,7 mm à une largeur d'épaulement de la pince de compression (12).

3. L'applicateur selon la revendication 1 ou 2, **caractérisé en ce que** le levier de fermeture (3) est pourvu d'une extrémité coudée (4) opposée à la seconde broche de fixation (2b).

4. L'applicateur selon la revendication 3, **caractérisé en ce que** la déviation de l'extrémité coudée (4) est de 20° par rapport à l'axe longitudinal de l'applicateur lorsque le levier de fermeture (3) est dans une position fermée.

5. L'applicateur selon l'une quelconque des revendications précédentes 1 à 4, **caractérisé en ce que** la poignée (5) est pourvue d'une partie plate (6).

6. L'applicateur selon l'une quelconque des revendications précédentes 1 à 5, **caractérisé en ce que** la poignée (5) se termine par une plaque (8).

7. Un kit de pinces de compression pour anastomoses orthopédiques comprenant un applicateur pour la mise en place de pinces de compression selon l'une quelconque des revendications précédentes 1 à 6, et une pince de compression (12) qui est fixée de manière amovible dans la rainure (10) de manière interchangeable avec le viseur (11).

8. Le kit selon la revendication 7, **caractérisé en ce que** la pince de compression (12) est composée d'une base à profil bas (13) et de bras verticaux (14).

9. Le kit selon la revendication 8, **caractérisé en ce que** les bras verticaux (14) présentent des extrémités pointues.
